Europäisches Patentamt

(19) European Patent Office

Office européen des brevets

(11) EP 0 477 143 B1

(12) **EUROPEAN PATENT SPECIFICATION**

(45) Date of publication and mention
of the grant of the patent:
**17.04.1996 Bulletin 1996/16**

(51) Int. Cl.⁶: **A61K 38/44**, A61K 47/34
// (A61K38/44, 31:71)

(21) Application number: **91830353.8**

(22) Date of filing: **21.08.1991**

(54) **Pharmaceutical composition having a cicatrising effect**

Vernarbungsfördernde pharmazeutische Verbindung

Composition pharmaceutique ayant en effect cicatrisant

(84) Designated Contracting States:
**AT BE CH DE DK ES FR GB GR LI NL SE**

(30) Priority: **30.08.1990 IT 4824490**

(43) Date of publication of application:
**25.03.1992 Bulletin 1992/13**

(73) Proprietor: **IDI FARMACEUTICI S.P.A.**
**I-00040 Pomezia RM (IT)**

(72) Inventors:
- **Guarnieri, Decimo**
**I-00040 Pomezia RM (IT)**
- **Fileccia, Piera**
**I-00168 Roma RM (IT)**

(74) Representative: **Bazzichelli, Alfredo et al**
**c/o Società Italiana Brevetti S.p.A.**
**Piazza di Pietra, 39**
**I-00186 Roma (IT)**

(56) References cited:
**FR-M- 11 18M** **GB-A- 1 056 192**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.11.2/3.4

## Description

The present invention refers to a pharmaceutical composition having a cicatrising effect which comprises as an active agent a therapeutically effective quantity of catalase and a pharmaceutically compatible vehicle in the form of a gel.

Prior Art

The problem of restoring the integrity of the skin after a wide variety of traumatisms is one with which any doctor is frequently faced. In spite of the fact that these traumatisms are often easily cured, there is a possibility of complications arising, depending on the site of the wound, on the patient in question, on any possible reinfection and on difficulty in cicatrisation.

Among the many remedies for said traumatisms is known to the state of the art the use of substances capable of promoting and aiding cicatrisation of the wound which caused the break in skin continuity, by means of stimulation of the proliferation of collagen.

Among those known to the state of the art, particular mention must be made of catalase, an enzyme found in all biological cells, which catalyses the decomposition of hydrogen peroxide in oxygen and water according to the following formula::

$$2H_2O_2 \rightarrow 2H_2O + O_2$$

The mechanism of action of catalase is substantially connected to the oxygen formed, which has a dual function: on the one hand it reaches the wounded tissues, stimulates their metabolism and local vascularisation, improving trophism and rendering cicatrisation easier, on the other hand, oxygen is active in preventing the survival of anaerobic bacteria, that is to say by blocking their vital functions. To this group belong the bacteria of the species Propioni-bacterium, which are already to be found on the normal skin, and other species, which can enter the wound, and which above all are extremely difficult to eradicate using common antibiotic treatments.

In spite of fact that the use of catalase as a cicatrising agent is known to the state of the art, a number of problems have arisen to prevent its use on an industrial level. The most important of these is the lability of catalase and the difficulty found in handling it. In the prior art is described a powder formulation, which is held to be the best for preventing or delaying degradation of the catalase: this formulation was distributed using an aerosol spray which also had the function of avoiding contact with the air, which causes disactivation of the enzyme.

However, it has been found that this formulation also shows problems in application.

Among these, the main ones are:

1) the difficulty in using nebulising gasses obtained from mixtures of dichlorodifluoromethane and tetrafluorodichloroethane because of their repercussions on the ozone layer;
2) obstruction of the distribution system caused by the particles of powder;
3) the strong sense of cold caused by the propellant gas when sprayed onto the wound, which is rendered even more unpleasant because used in an inflamed area;
4) the difficulty in estimating the exact quantity of the dose sprayed;
5) the formation of a crust consequent to the application of a powder preparation, which renders the underlying layers of the wound impermeable to treatment, and which nullifies, or greatly reduces, the positive effects of the substance applied.

It has now been suprisingly found that the use of a catalase-based formulation in a gel vehicle makes it possible to overcome the problems found in the prior art.

Subject of the invention

Subject of the present invention is therefore a pharmaceutical composition having a cicatrising effect, comprising as an active agent a therapeutically effective amount of catalase together with a pharmaceutically compatible vehicle, characterised by the fact that said vehicle is present in the form of a gel.

Administration of catalase by means of a watery gel, as a topical preparation, shows several advantages, among which the following can be mentioned:

1) as it is extremely rich in water, the gel allows transport of hydrosoluble substances and, due to the slow evaporation of the water content, enables the skin wounds to be reabsorbed slowly without excessive de-hydration of the epidermis;

2) thanks to the presence of a polymer which holds back water, evaporation is extremely slow, so that a good distribution of active principle/excipients/skin is guaranteed and maintained over a period of time;

3) the formulation is extremely stable, this being an element of great importance for a delicate substance such as catalase.

The addition to the formulation containing catalase in a gel vehicle of a wide-spectrum antibiotic, such as gentamicin, also makes it possible to improve the kinetics of cicatrisation, coupling to the cicatrising effect of catalase the sterilising capacity typical of gentamicine, which is a bactericide for most Gram positive and Gram negative germs.

Because of the low stability of catalase in air, it is important to minimise or eliminate altogether any oxygen-catalase contacts, which would cause oxydation and, therefore, disactivation of the enzyme. For this purpose it has been shown to be particularly advantageous the use of tubes commercially known as Precitubs (manufactured by the firm Tubopak of Tortona), which are small polyethylene tubes which mount a Valois distribution pump without air inlet. An advantage given by the use of these tubes is that of making possible the distribution of a known quantity of the product to be consumed. However, the use of the above indicated tubes should not be considered to limit the scope of the present invention, as the composition can also be applied by means of dosers/distributors known to the state of the art.

From an applicative point of view, furthermore, the gel formulation is extremely useful, because:

- it disperses the active principle very well:
- avoids effects of maceration, occlusion and excessive dehydration with the formation of crusts, which impede the cicatrisation of the wounded tissues and alter the regular trophism of the areas close to the lesions;
- it absorbs, because of its polimer content, exuded substances, thus contributing towards cleaning the wounds;
- the formulation containing gentamicine, which is a bactericidal agent for the majority of Gram positive and Gram negative germs, makes possible the thorough and rapid sterilisation of infected wounds, explicating its beneficient effect above all where there are evident clinical and microbiological signs of the presence of pathogenic bacteria.

From a therapeutic point of view the product is indicated for use with ulcers, wounds and burns to facilitate the cleaning process (type containing antibiotic) and cicatrisation of wounds (type not containing antibiotic); to all this is constantly added the anti-anaerobic activity typical of catalase, which is a known oxydising agent.

Particularly preferred formulations of the pharmaceutical composition having a cicatrising effect according to the present invention are the following:

| Cicatrising formulation | % by weight |
| --- | --- |
| Catalase with reference to the total weight of the composition | 0.2 - 0.3 |
| Propylenic glycol | 4.0 - 6.0 |
| Propyl paraoxybenzoate | 0.02 - 0.03 |
| Methyl paraoxybenzoate | 0.02 - 0.03 |
| Sodium salt of acrylic acid and acrylamide copolymer | 0.4 - 0.6 |
| Distilled water | 93.0 - 95.0 |

| Detergent formulation with antibiotic | % by weight |
|---|---|
| Catalase | 0.2 - 0.3 |
| Gentamicin | 0.1 - 0.2 |
| Propylen glycol | 4.0 - 6.0 |
| Methyl paraoxybenzoate | 0.02 - 0.03 |
| Propyl paraoxybenzoate | 0.2 - 0.03 |
| Sodium salt of acrylic acid and acrylamide copolymer | 0.5 - 1.5 |
| Water | 93.0 - 95.0 |

As regards the starting products, it is preferred to use equine catalase at 2,850,000 UI/g; but other types of catalase can also be advantageously used; the thickeners for the formation of the gel, gentamicin and the other vehicles are products well known to the state of the art.

In the following are listed two particularly preferred examples of compositions according to the present invention, with reference to 100 g of gel.

Example 1

| Active principle | |
|---|---|
| Equine catalase 2,850,000 IU/g (Lab. Zanoni, Milan) | 800,000 IU |
| Excipients | |
| Propylenic glycol (Romana Chimici, Anagni FR | 5.0 g |
| Propyl para-oxybenzoate (Fumagalli, Rome) | 0.025 g |
| Methyl para-oxybenzoate (Fumagalli, Rome) | 0.025 g |
| Sodium salt of acrylic acid and acrylamide copolymer (Hoechst Italiana, Rome) | 0.5 g |
| Distilled water | to make up 100.0 g |

Example 2

| Active principles | |
|---|---|
| Equine catalase 2,850,000 IU/g (Lab. Zanoni, Milan) | 800,000 IU |
| Gentamicin sulphate (CEM - MI) | 0.166 g |
| Excipients | |
| Propylenic glycol (Romana Chimici, Anagni FR) | 5.0 g |
| Propyl para-oxybenzoate (Fumagalli, Rome) | 0.025 g |
| Methyl p-oxybenzoate (Fumagalli, Rome) | 0.025 g |
| Sodium salt of acrylic acid and acrylamide copolymer (Hoechst Italiana, Rome) | 1.0 g |
| Distilled water | to make up 100.0 g |

Example of production of a pharmaceutical composition according to the present invention.

The first phase consists in the weighing of an exact quantity of sterile distilled water, which will serve to bring the final volume up to 100 ml. The two solutions described herebelow are then prepared.

| | | |
|---|---|---|
| 1. | Propylenic glycol | 5.0 g |
| | Methyl para-oxybenzoate | 0.025 g |
| | Propyl para-oxybenzoate | 0.025 g |
| 2. | Equine catalase 2,850,000 IU/g | 800,000 IU |
| | sterile distilled water | 10.0 g |
| | Gentamicine sulphate (in the case of the version containing antibiotic) | 0.166 g |

the solutions are added one after the other to the water, the mixture is then agitated to render it homogeneous; at this point the powder is added under strong stirring:

| | |
|---|---|
| Sodium salt of acrylic acid and acrylamide copolymer | 1.0 g |

continuing to stir until it is completely dispersed and the product is homogeneous.

The gel is then left under vacuum until all the air has been removed from the product, and then put into the dose dispensers ready for application.

**Claims**

1. Pharmaceutical composition having a cicatrising effect, comprising as an active agent a therapeutically effective amount of catalase together with a pharmaceutically compatible vehicle, characterised by the fact that said vehicle is present in gel form.

2. Composition according to claim 1, further comprising the antibiotic gentamicin or its pharmaceutically tolerable salts.

**3.** Composition according to claim 1, comprising:

| catalase | 0.2 - 0.3 % |
|---|---|
| propylenic glycol | 4.0 - 6.0 % |
| methyl paraoxybenzoate | 0.02 - 0.03 % |
| propyl paraoxybenzoate | 0.02 - 0.03 % |
| sodium salt of acrylic acid and acrylamide copolymer | 0.4 - 0.6 % |
| water | 93.0 - 95.0 % |

all percentuals being with reference to the total weight of the composition.

**4.** Composition according to claim 2, comprising:

| catalase | 0.2 - 0.3 % |
|---|---|
| gentamicin sulphate | 0.1 - 0.2 % |
| propylenic glycol | 4.0 - 6.0 % |
| propyl paraoxybenzoate | 0.02 - 0.03 % |
| methyl paraoxybenzoate | 0.02 - 0.03 % |
| sodium salt of acrylic acid and acrylamide copolymer | 0.5 - 1.5 % |
| water | 93.0 - 95.0 % |

all percentuals being with reference to the total weight of the composition.

**5.** Pharmaceutical composition as claimed in claims 1 to 4, characterised by the fact that the catalase employed is equine catalase.

**Patentansprüche**

**1.** Pharmazeutische Zusammensetzung mit einer narbenbildenden Wirkung, umfassend als ein aktives Mittel eine therapeutisch wirksame Menge an Katalase zusammen mit einem pharmazeutisch verträglichen Vehikulum, **dadurch gekennzeichnet, daß** das Vehikulum in Gel-Form vorliegt.

**2.** Zusammensetzung nach Anspruch 1, ferner umfassend das Antibiotikum Gentamicin oder seine pharmazeutisch zulässigen Salze.

**3.** Zusammensetzung nach Anspruch 1, umfassend:

| Katalase | 0,2 bis 0,3 % |
|---|---|
| Propylenglykol | 4,0 bis 6,0 % |
| Methylparaoxybenzoat | 0,02 bis 0,03 % |
| Propylparaoxybenzoat | 0,02 bis 0,03 % |
| Copolymer aus Natriumsalz der Acrylsäure und Acrylamid | 0,4 bis 0,6 % |
| Wasser | 93,0 bis 95,0 % |

wobei alle Prozentsätze auf das Gesamtgewicht der Zusammensetzung bezogen sind.

4. Zusammensetzung nach Anspruch 2, umfassend:

| Katalase | 0,2 bis 0,3 % |
|---|---|
| Gentamicinsulfat | 0,1 bis 0,2 % |
| Propylenglykol | 4,0 bis 6,0 % |
| Methylparaoxybenzoat | 0,02 bis 0,03 % |
| Propylparaoxybenzoat | 0,02 bis 0,03 % |
| Copolymer aus Natriumsalz der Acrylsäure und Acrylamid | 0,5 bis 1,5 % |
| Wasser | 93,0 bis 95,0 % |

wobei alle Prozentsätze auf das Gesamtgewicht der Zusammensetzung bezogen sind.

5. Pharmazeutische Zusammensetzung nach einem der Ansprüche 1 bis 4,
   **dadurch gekennzeichnet, daß**
   die verwendete Katalase Pferdekatalase ist.

**Revendications**

1. Composition pharmaceutique ayant un effet cicatrisant, comprenant comme agent actif une quantité thérapeutiquement efficace de catalase ainsi qu'un véhicule pharmaceutiquement compatible, caractérisée par le fait que ledit véhicule est présent sous forme de gel.

2. Composition selon la revendication 1, comprenant en outre l'antibiotique gentamycine ou ses sels pharmaceutiquement tolérables.

3. Composition selon la revendication 1, comprenant:

| catalase | 0,2 - 0,3 % |
|---|---|
| propylèneglycol | 4,0 - 6,0 % |
| paraoxybenzoate de méthyle | 0,02 - 0,03 % |
| paraoxybenzoate de propyle | 0,02 - 0,03 % |
| copolymère de sel de sodium d'acide acrylique et d'acrylamide | 0,4 - 0,6 % |
| eau | 93,0 - 95,0 % |

tous les pourcentages étant rapportés à la masse totale de la composition.

4. Composition selon la revendication 2, comprenant:

| | |
|---|---|
| catalase | 0,2 - 0,3 % |
| sulfate de gentamycine | 0,1 - 0,2 % |
| propylèneglycol | 4,0 - 6,0 % |
| paraoxybenzoate de propyle | 0,02 - 0,03 % |
| paraoxybenzoate de méthyle | 0,02 - 0,03 % |
| copolymère de sel de sodium d'acide acrylique et d'acrylamide | 0,5 - 1,5 % |
| eau | 93,0 - 95,0 % |

tous les pourcentages étant rapportés à la masse totale de la composition.

5. Composition pharmaceutique selon les revendications 1 à 4, caractérisée par le fait que la catalase employée est la catalase équine.